# EUROPEAN PATENT APPLICATION

(11) **EP 4 841 643 A1**
(43) Date of publication of application: **18.11.2026**
(21) Application number: 25175651.6
(22) Date of filing: 12.05.2025
(51) Int. Cl.: G16H 50/20

(54) **MEDICAL IMAGE PREDICTION METHOD AND SYSTEM**

(71) Applicant: Koninklijke Philips N.V., 5656 AE Eindhoven (NL)
(72) Inventor: GRASS, Michael, Eindhoven (NL); VON BERG, Jens, Eindhoven (NL); FORTHMANN, Peter, 5656AG Eindhoven (NL); WEIß, Steffen, Eindhoven (NL); VOGTMEIER, Gereon, Eindhoven (NL)
(74) Representative: Philips Intellectual Property & Standards

(57) **Abstract**

A computer-implemented method for predicting medical and/or optical images is provided. The method includes receiving a medical image (72) of a patient's anatomy and an optical image (74) of an exterior area of the patient's body. These images are input into an image prediction module (80) comprising a trained prediction model. The module outputs either or both of a prediction medical image (22) representing a predicted appearance of the anatomy and a prediction optical image (24) representing a predicted appearance of the exterior body area. The prediction images may represent future appearances or disease progression. The method enables synergistic processing of complementary medical and optical image data to generate more accurate predictions of anatomical and external changes over time.

## Description

### FIELD OF THE INVENTION

The present invention relates to the field of AI-enabled medical image processing systems for diagnostic support.

### BACKGROUND OF THE INVENTION

Machine vision models have demonstrated capabilities in predicting future appearances of individuals from photographs. This technology has potential applications in the medical field. Projecting medical images of patient anatomy into the future could provide valuable insights for clinicians. Such projections could allow visualization of potential anatomical changes over time.

The medical domain presents unique challenges for image prediction. Anatomies with pathologies involve numerous variables affecting future appearances. These variables exceed those considered in facial aging predictions.

### SUMMARY OF THE INVENTION

It is the recognition of the inventors that accurate forward-projection of medical images benefits from incorporation of additional information.

The invention is defined by the claims.

An aspect of the invention is a computer-implemented method. The method comprises: receiving a medical image of an anatomy of a patient and receiving an optical image of an exterior area of the patient's body. The method further comprises inputting the medical image and the optical image into an image prediction module comprising a trained prediction model, and, responsive to which, outputting from the image prediction module either or both of: a prediction medical image representing a predicted appearance of the anatomy; and a prediction optical image representing a predicted appearance of the exterior area of the patient's body.

Embodiments of the invention are based on the recognition that prediction image generation of an anatomy may be enhanced by incorporating additional information sources. One convenient and useful source of information is an optical image of the patient's exterior body area. This optical image may be acquired using a camera.

The appearance of the body's exterior is correlated to the appearance and structure of internal anatomy. This correlation is particularly relevant for predicting future anatomical appearances. The relationship between external and internal features becomes more pronounced in the presence of pathologies.

Pathologies may for example impact both the primary affected anatomy and visible external body properties. Similarly, external traumas may have effects on internal pathologies and their progression. Integrating optical image information into the input dataset for the prediction model may therefore enhance accuracy. Optical images are simple and convenient to obtain, often simultaneously with medical image acquisition.

Some medical imaging modalities already incorporate optical imaging systems. For example, x-ray imaging systems often use optical cameras to align the field of view. Tomographic imaging systems, such as MRI or CT, employ multiple cameras to detect patient positioning and other factors, and plan the imaging workflow. Utilizing these existing optical imaging capabilities provides a highly convenient method for obtaining additional input data.

The optical image data can serve as a valuable input to the model for predicting future anatomical appearances in medical images. This approach leverages readily available technology to enhance prediction accuracy. The simultaneous acquisition of medical and optical images ensures temporal consistency between the datasets.

Integrating optical and medical image data allows the prediction model to consider both internal and external factors which may capture subtle indicators of anatomical changes not visible in medical images alone.

In some embodiments, the received medical image and received optical image may be images acquired concurrently during the same imaging session.

As used herein, the term "imaging session" may refer to a period of time during which one or more imaging procedures are performed on a patient. An imaging session typically involves the patient being positioned for imaging and may include the acquisition of one or more images using one or more imaging modalities. The imaging session may be continuous or may include brief interruptions for patient repositioning or equipment adjustments. Multiple images acquired during an imaging session are generally considered to represent the patient's condition at substantially the same point in time.

In some embodiments, the medical image may depict an anatomy having a pathology. The prediction medical image may be an image of the anatomy with the pathology at a later stage of progression.

In some embodiments, the exterior area of the patient's body corresponds to an exterior of an area of the body which contains the said anatomy. This alignment between the medical and optical images allows for correlation of internal anatomical features with external visible indicators.

In other words, the optical image field of view, FOV, may represent an outside body surface overlying what is imaged with the medical image. In other words, the FOV of the optical image represents the outside body surface corresponding to the region imaged by the medical imaging modality. For example, when using x-ray imaging, the optical image captures the external surface area above the internal structures visible in the x-ray.

This alignment allows the optical image to provide information about surface-visible indications related to the internal anatomy. Such indications may include scarring, deformation, trauma, or other external manifestations at the site of an internal pathology, e.g. bandages or fixations.

In some embodiments, the trained prediction model is trained to receive as inputs the first image and the second image and to generate as an output the third image.

In some embodiments, the anatomy of the patient represented in the received medical image is characterized by a pathology at a first stage of progression. The optical image may be an image acquired at a time when the pathology of the patient is at the first stage of progression.

In some embodiments, the prediction medical image represents a predicted appearance of said anatomy of the patient with the pathology at a later stage of progression; and/or the prediction optical image represents a predicted appearance of the exterior area of the patient's body with the pathology at a later stage of progression. This enables tracking and prediction of disease progression over time.

A model suitable for realizing this may be trained and configured in different ways. In some embodiments, the particular future progression stage of the pathology which is to be represented in the future image may be pre-defined, e.g. the prediction model is trained to generate prediction images corresponding to a pre-defined grade or progression stage of a pre-defined pathology responsive to an input image. This allows for visualization of expected disease progression, aiding in treatment planning. However, in other embodiments, the model may incorporate a variable parameter which can be configured by a user to adjust the future disease progression stage which is to be represented in the future image.

In some embodiments, the prediction medical image represents a predicted appearance of said anatomy of the patient at a later (future) point in time; and/or the prediction optical image represents a predicted appearance of the exterior area of the patient's body at a later point in time. In some embodiments, the image prediction model may be trained so that the later point in time corresponds to a pre-defined temporal offset from an acquisition time associated with the medical image and/or the optical image, e.g. five years into the future, or ten years into the future. However, in other embodiments, the image prediction model may incorporate a variable parameter which can be configured by a user to adjust the temporal offset which is to be applied in generating the prediction image, e.g. the user can choose to generate a prediction image which represents a future point 5 years or 10 years into the future.

In some embodiments, the prediction model may be trained to generate prediction images which represent a predicted appearance of the anatomy or the exterior area of the patient's body
a pre-defined time interval into the future; and/or
with a pre-defined pathology of the patient at a predefined stage of progression.

In some embodiments, the image prediction module comprises a feature extraction layer configured to process the received optical image and extract a predetermined set of one or more features from the image, and wherein the trained image prediction model is configured to process the received medical image and the set of extracted features and to generate as an output the prediction medical image or prediction optical image. In other words, the input to the model is a set of one or more features extracted from the optical image. By way of example, the set of one or more features extracted from the optical image may include, but are not limited to, skin features, posture, and pose. Additional features that may be extracted may comprise body mass index (BMI) indicators, visible signs of aging, and external manifestations of medical conditions. Skin features that may be extracted may include texture, color variations, and the presence of lesions or other dermatological conditions. Posture-related features may encompass spinal alignment, shoulder positioning, and overall body stance. Pose features may include limb positioning and body orientation relative to the imaging plane.

By utilizing extracted features rather than raw pixel data from the optical image, the model's input dimensionality may be reduced. This reduction may lead to improved computational efficiency.

In some embodiments, the model may be trained to additionally receive as input non-imaging medical information associated with the patient to which the input medical image and input optical image correspond, such as medical history information and/or values for one or more clinical parameters. The values of one or more clinical parameters may include values for one or more vital signs.

Examples of non-imaging medical information which the model may be trained to receive as input may include one or more of: heart disease status, diabetes status, osteoporosis status, bone density, BMI, osteoarthritis status.

In some embodiments, the imaging apparatus used to acquire the medical image, and the optical image may include a physiological parameter measurement system for measuring one of more physiological parameters, for example including one or more vital signs, while the medical and optical images are being acquired, during the imaging session. The image prediction model may be trained to receive values for the measured one or more physiological parameters as further input in addition to the medical image and the optical image.

In some embodiments, the image prediction model comprises a generative adversarial network (GAN). GANs consist of two neural networks: a generator and a discriminator. These compete against each other during training. The use of GANs enables the generation of highly realistic prediction images. In some embodiments, the image prediction model may be trained using a cycle-GAN operation applied to a training data set. Cycle-GANs are particularly effective for maintaining consistency between input and output image domains. This helps in preserving anatomical structure in medical image predictions.

A particular advantage of cycle-GAN training is that it does not require paired input-output training images. Instead, it utilizes unpaired images from two different domains. This characteristic is particularly valuable in medical imaging, where paired future state images are often unavailable. The cycle-GAN training process leverages cycle consistency loss to enforce structural coherence. This mechanism ensures that the generated images maintain fidelity to the original anatomical structures. The cycle consistency principle applies transformations in both forward and backward directions between domains.

In some embodiments, the cycle-GAN operation is applied using a training dataset comprising a plurality of training image pairs, wherein each training image pair comprises a medical image of said anatomy of a patient and an optical image of the exterior area of the body of the same patient, and wherein the medical image and optical image are images acquired concurrently in a same imaging session. Concurrent acquisition ensures temporal consistency between the internal anatomical representation and the external body appearance.

In some embodiments, the plurality of training image pairs include image pairs corresponding to patients of different ages and/or at different progressions of pathology of the anatomy. This diverse training dataset allows the model to learn and predict a wide range of age-related changes and disease progressions.

In some embodiments, the trained prediction model is a multi-channel model having a first input channel and a second input channel, and wherein the first input channel is configured to receive and process data corresponding to the optical image of the exterior area of the patient's body, and the second input channel is configured to receive and process data corresponding to the medical image of the anatomy.

In some embodiments, the trained prediction model may include at least a third input channel configured to receive and process input non-imaging medical information associated with the patient to which the input medical image and input optical image correspond, such as medical history information and/or values for one or more clinical parameters. The values of one or more clinical parameters may include values for one or more vital signs. Further examples of non-imaging medical information which the model may be trained to receive in the optional third input channel as input may include one or more of: pose, weight, age, BMI, blood pressure, injuries, pathologies, nutrition, laboratory values, prosthetics, cardiac and respiratory cycle information, heart disease status, diabetes status, osteoporosis status, bone density, and osteoarthritis status.

A fourth, fifth and further channels may also be added depending on desired inputs and system configuration.

In some embodiments, the received medical image is any one of: an x-ray image, a computed tomography (CT) image, a magnetic resonance (MR) image, or an ultrasound image.

In some embodiments, the predicted medical image depicts a predicted appearance of an image of the anatomy captured with an imaging modality which is the same as the imaging modality of the received medical image.

The predicted medical image may depict a predicted appearance of an x-ray image of the anatomy in some embodiments.

In some embodiments, the method comprises controlling a medical imaging system to acquire the medical image of the anatomy of the patient, and concurrently controlling an optical imaging system to acquire the optical image of the exterior area of the body of the patient. This integrated acquisition approach ensures temporal and spatial correlation between the medical and optical images.

In one set of embodiments, the medical imaging system is an x-ray imaging system, and the medical image is an x-ray image.

The optical imaging system may comprise a camera.

A further aspect of the invention is a computer program product. The computer program product comprises computer program code configured, when run on a processor, to perform a method in accordance with any embodiment described in this disclosure or in accordance with any claim.

A further aspect of the invention is a processing device. The processing device comprises one or more processors configured to perform a method in accordance with any embodiment described in this disclosure or in accordance with any claim.

A further aspect of the invention is a system. The system comprises an imaging apparatus. The imaging apparatus comprises: a medical imaging system, an optical imaging system and an imaging controller operatively coupled to the medical imaging system and the optical imaging system. The imaging controller is configured to control acquisition of a medical image of the anatomy of the patient and an optical image of the exterior area of the body of the patient during a same imaging session. The system further comprises a processing device in accordance with any embodiment described in this disclosure or in accordance with any claim.

In some embodiments, the medical imaging system is arranged to image a first imaging region and the optical imaging system is arranged to image a second imaging region, and wherein the first imaging region and second imaging region are overlapping or identical. This configuration ensures spatial correlation between the medical and optical images, enhancing the accuracy of the prediction model.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example computer-implemented method in accordance with one or more embodiments of the invention;
Fig. 2 depicts a block diagram of a system in accordance with one or more embodiments of the invention;
Fig. 3 shows a block diagram schematically illustrating a processing flow in accordance with one or more embodiments of the invention;
Fig. 4 depicts a sample cycle-GAN image prediction model in accordance with one or more embodiments;
Fig. 5A depicts a sample generator for a multi-channel cycle GAN;
Fig. 5B depicts a sample discriminator for a multi-channel cycle GAN; and
Fig. 6 depicts sample training data in accordance with one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Embodiments of the invention provide a computer-implemented method for predicting medical and/or optical images. The method comprises receiving a medical image of a patient anatomy and an optical image of an exterior area of the patient's body. These images are input into an image prediction module comprising a trained prediction model. The module outputs either or both of a prediction medical image representing a predicted appearance of the anatomy and a prediction optical image representing a predicted appearance of the exterior body area. The prediction images may represent future appearances or disease progression. The method enables synergistic processing of complementary medical and optical image data to generate more accurate predictions of anatomical and external changes over time.

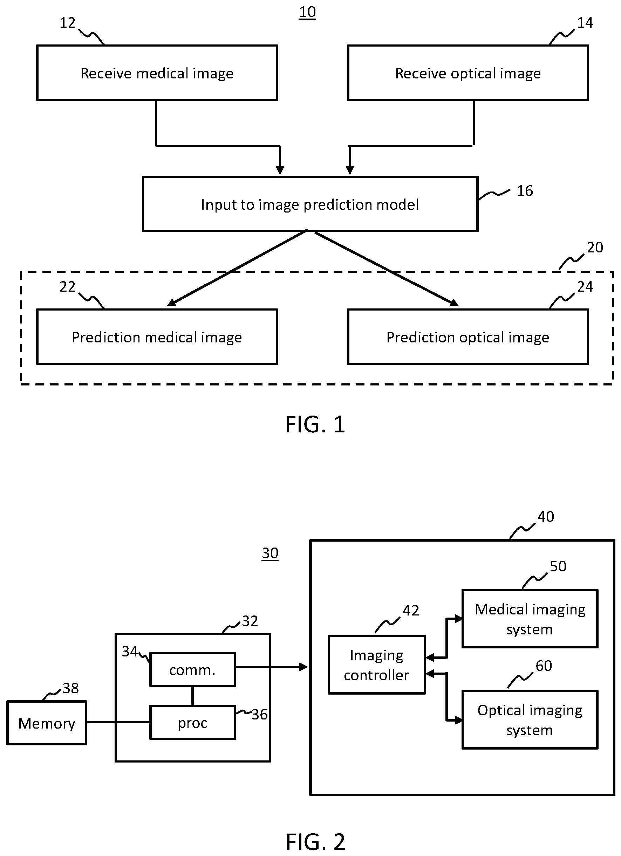

Fig. 1 outlines steps of an example computer implemented method in accordance with one or more embodiments of the invention.

The method 10 comprises receiving 12 a medical image of an anatomy of a patient. The medical image may correspond to an imaging modality for capturing internal anatomical structures of the patient. By way of non-limiting example, the received medical image may comprise any one or more of: an x-ray image, a computed tomography (CT) image, a magnetic resonance (MR) image, or an ultrasound image.

The method 10 further comprises receiving 14 an optical image of an exterior area of the same patient's body. By way of example, the optical image may be captured using an optical imaging device. The optical image may represent the external appearance of a portion of the patient's body.

In some embodiments, the exterior area of the patient's body captured in the optical image may correspond to an exterior of an area of the body which contains the anatomy depicted in the medical image. In other words, the optical image field-of-view (FOV) represents an outside body surface overlying what is imaged within the body in the medical image. In this way, by way of example, if the imaged anatomy in the medical image is pathological, the second image may provide indications of scarring, trauma, or other surface-visible indications of the pathology.

The method 10 further comprises inputting 16 the medical image and the optical image into an image prediction module comprising a trained prediction model. The image prediction module is configured to process both the medical image data and the optical image data and, responsive to this, output 20 one or more prediction images.

The step of outputting 20 the one or more prediction images from the image prediction module may comprise outputting either or both of: a prediction medical image 22 representing a predicted appearance of the anatomy (represented in the original input medical image), and a prediction optical image 24 representing a predicted appearance of the exterior area of the patient's body.

The prediction medical image 22 may provide a predicted visualization of how the internal anatomical structures captured in the original medical image would look at a different time point, offset from the time of acquisition of the input images, or if the anatomy or the patient were subject to a pathology at a particular stage of progression. If the imaged anatomy already comprises a pathology, the prediction medical image may represent a predicted appearance of the anatomy with the pathology at a different stage of progression or a different disease grade.

The predicted medical image may depict a predicted appearance of an image of the anatomy captured with an imaging modality which is the same as the imaging modality of the received medical image. However, this is not essential in the case that the prediction model is trained to generate prediction images predicting appearance of images of a different imaging modality.

The one or more prediction images may represent an appearance of the anatomy or the outside of the body at a future point in time from the time of acquisition of the input images, for example 5 or 10 years into the future, but this is not essential. A prediction model may equally be trained to predict an earlier time, and this may be useful for example for generating synthetic training data for training one or more other models.

Additionally or alternatively, the prediction images may provide a visualization of a predicted appearance of the anatomy or outside of the body with a pathology of the patient at a future or more advanced disease state, but again this is not essential.

Another aspect of the invention is a processing device comprising one or more processors configured to perform the steps of the method 10. The processing device may be configured to receive the medical image and optical image data, input the data into the image prediction module, and output the resulting prediction images.

Another aspect of the invention is a computer program product comprising computer program code configured to perform the described steps when run on a processor.

Fig. 2 shows an example system 30 in accordance with one or more embodiments of the invention.

The system 30 comprises a processing device 32 that includes one or more processors 36. The one or more processors 36 may be configured to perform a method in accordance with the method illustrated in Fig. 1, or with any embodiment described in the present disclosure or in accordance with any claim.

The processing device 32 further comprises a communication module or input/output 34. The communication module or input/output 34 is configured to enable communication with external devices, including receiving and outputting data.

The system 30 may additionally comprise a memory 38. The memory 38 is configured to store computer program code, such as machine-executable instructions. The stored instructions may be executed by the one or more processors 36 to perform a method in accordance with any embodiment described in the present disclosure. The computer program code may form a computer program product.

In the illustrated example, the system 30 further comprises an imaging apparatus 40. The imaging apparatus 40 comprises, in the illustrated example, a medical imaging system 50, an optical imaging system 60, and an imaging controller 42. The imaging controller 42 is operatively coupled to both the medical imaging system 50 and the optical imaging system 60.

The imaging controller 42 may be configured to control acquisition of a medical image of an anatomical region of the patient and an optical image of an exterior area of the patient's body. In some examples, the imaging controller 42 may be configured to control acquisition of both images during a single imaging session.

In the illustrated example, the communication module 34 of the processing device 32 is operatively or communicatively coupled to the imaging controller 42. This coupling enables transfer of medical imaging data and optical imaging data from the imaging controller 42 to the processing device 32.

With regard to the medical imaging system 50, by way of non-limiting example, this may comprise any one or more of: an x-ray imaging system, a computed tomography (CT) imaging system, a magnetic resonance (MR) imaging system, and an ultrasound imaging system.

Regarding the optical imaging system 60, by way of non-limiting example, this may comprise one or more cameras. It may comprise a video camera in some examples. By way of example, it is common in x-ray imaging setups to provide an imaging environment that comprises the x-ray imaging apparatus in combination with one or more optical modality video imaging devices used for the purpose of monitoring patient positioning relative to the x-ray imaging apparatus. This optical image data is inherently produced contemporaneously with the x-ray images of the x-ray imaging examination. It is the recognition of the inventor that these might be used as a supplementary input the prediction model for improving accuracy of output prediction images.

Likewise, beyond the x-ray imaging modality, it is very common to use optical imaging devices to obtain video imagery of patients, e.g., in MRI scanning rooms or CT scanning rooms, for the purpose of monitoring patient status and/or for purposes of guiding or determining the positioning the patient relative to the medical imaging equipment. Such optical image data may likewise be used as the input to the prediction model since it naturally provides a contemporaneous complement to the medical imagery acquired during the imaging examination.

The optical imaging system may comprise any variety of optical imaging device. The optical imaging system 60 may comprise a camera or other optical sensor capable of capturing visible light images. The optical imaging system 60 is configured to acquire the optical image 74 of the external surface of the patient's body. The optical image can be a single image frame or a video clip for example.

In some embodiments, the imaging apparatus 40 may define a spatial arrangement of the medical imaging system 50 and the optical imaging system 60. For example the two may have a fixed mounting position relative to one another. In some embodiments, at least the optical imaging system 60 may comprise an imaging field of view (FOV) which is adjustable or configurable. In examples, the optical and medical image may be aligned or correlated with each other by use of image alignment or correlation techniques.

To ensure correspondence between the medical images and the optical image, in some embodiments, the medical imaging system 50 may be arranged to image a first imaging region and the optical imaging system 60 is arranged to image a second imaging region, and wherein the first imaging region and second imaging region are overlapping or identical. In other words, the FOV of the two may overlap or be identical. This has the result of obtaining optical images with an optical image FOV which represents an outside body surface overlying what is imaged with the medical image (e.g. an x-ray image).

In some embodiments, the optical imaging system 60 has an adjustable FOV, and wherein the imaging controller 42 is operable to and is configured to adjust a FOV of the optical imaging system 60 so that the imaging region (FOV) which the optical imaging system 60 is arranged to image overlaps or is identical with an imaging region (FOV) which the medical imaging system 50 is arranged to image.

In some embodiments, the imaging controller 42 is configured to use an optical image output of the optical imaging system 60 to adjust a positioning of a medical imaging field-of-view of the medical imaging system 50 relative to a position of the patient prior to acquiring the medical image 72 using the medical imaging system 50. This may be a functionality already integrated in the imaging apparatus 40, e.g. the optical imaging system 60 is used for the purpose of guiding FOV positioning and planning relative to the patient anatomy.

The imaging controller 42 may be configured to manage the timing and synchronization of image acquisition of the medical imaging system 50 and the optical imaging system 60. By controlling both imaging systems concurrently, the imaging controller 42 enables capture of contemporaneous internal and external patient data. This concurrent acquisition provides temporally consistent image pairs for subsequent processing and analysis. However, the optical image may also be provided independently, for example from a mobile phone which is used to take the optical image during or right before or after the imaging session.

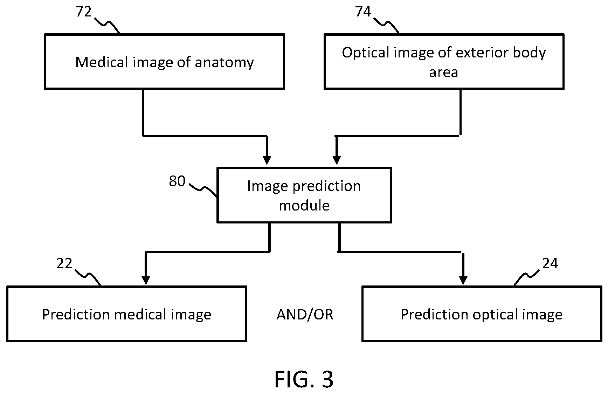

Fig. 3 schematically illustrates the processing flow and inputs/outputs of the image prediction model.

As schematically depicted, a received medical image 72 of an anatomy of a patient and a received optical image 74 of an exterior area of the patient's body are provided as two inputs to the image prediction module 80. The image prediction module comprises one or more trained prediction models which are configured to output one or both of: a prediction medical image 22 representing a predicted appearance of the anatomy, and a prediction optical image 24 representing a predicted appearance of the exterior area of the patient's body.

As already discussed above, the prediction image(s) may represent a predicted appearance of the anatomy and/or outside of the patient's body at a later time point or when a pathology or disease state of the anatomy or patient is at a more advanced stage.

One example application of the image prediction system may be the visualization of scoliosis progression over time. The system may be utilized to predict and illustrate changes in spinal alignment. A received medical image, such as an x-ray, may depict the patient's spine at an initial status, characterized by a mild lateral curvature, corresponding to a first stage of progression of the pathology. The prediction model may be trained to process this initial medical image, along with a corresponding optical image of the patient's exterior body area, to generate prediction images. An output prediction medical image may be produced representing a predicted appearance of the patient's spine at a later stage of scoliosis progression, for example after a seven-year period. This predicted image may exhibit a more pronounced curvature characteristic of advanced scoliosis. The generated prediction image may provide healthcare professionals with a visual representation of potential spinal curvature development over time, facilitating treatment planning and patient education.

Additionally or alternatively, in some embodiments, the image prediction module 80 may be configured to output a prediction optical image representing a predicted appearance of the exterior area of the patient's body with scoliosis at a later stage of progression. This prediction may visualize changes in the patient's posture, shoulder alignment, or other external indicators of spinal curvature that correspond to the internal progression of scoliosis.

By comparing the initial medical image to the prediction medical image, healthcare providers can assess the potential severity of scoliosis progression and plan appropriate interventions. The prediction optical image can complement this assessment by illustrating how the condition may manifest externally, aiding in patient education and treatment planning.

The scoliosis progression example demonstrates how the image prediction system can process medical and optical images characterized by a pathology at an initial stage to generate predictions of both internal anatomical changes and external physical manifestations at later stages of disease progression.

The provided system for medical image prediction may be advantageously applied for use in predicting advancement of various pathologies and the example relating to progression of spinal curvature in scoliosis is presented simply as one illustration of the general principle. Other examples comprise, in a non-limiting way, the progression of arthritis, progression of internal fluid build-up, progression/healing of fractures, progression of lung conditions such as cystic fibrosis, progression/growth of benign and non-benign tumors, progression of gallstones, progression of degenerative spinal conditions such as scoliosis or herniated discs, and progression of cardiovascular conditions such as atherosclerosis or aneurysms. Additionally, the method may be used to predict the healing of post-surgical sites, the development of diabetic complications such as foot ulcers, or the progression of liver diseases such as cirrhosis.

By way of example, the method may be used for diverse imaging modalities, for example:
X-ray imaging: Commonly used for monitoring the progression of arthritis, healing of fractures, and degenerative spinal conditions such as scoliosis. X-rays are also suitable for detecting calcifications in cardiovascular conditions like atherosclerosis.

Computed Tomography (CT): Ideal for assessing the progression of internal fluid build-up, such as pleural effusion or ascites, and for tracking the growth of tumors, both benign and malignant. CT is also frequently used for evaluating gallstones and aneurysms in the cardiovascular system.

Magnetic Resonance (MR) Imaging: Particularly effective for visualizing soft tissue changes, such as the progression of cystic fibrosis in the lungs, the development of herniated discs, or the progression of liver cirrhosis. MR is also valuable for monitoring the progression of brain tumors or neurological conditions.

Ultrasound Imaging: Often employed for tracking the progression of gallstones, internal fluid build-up such as ascites, and complications in pregnancy. Ultrasound is also useful for monitoring the healing of soft tissue injuries or post-surgical sites.

Regarding the trained prediction model, example implementation details will now be discussed in more detail.

In some embodiments, the trained prediction model may comprise one or more artificial neural networks. The trained prediction model may comprise one or more Convolutional Neural Networks (CNNs).

The trained prediction model is trained to receive as input the medical image and the optical image and to generate as output at least one of: a prediction medical image and a prediction optical image.

For purposes of the present description, an example will be considered in which the input to the prediction model comprises the medical image of the anatomy and the optical image of the area of the outside of the body, acquired contemporaneously, e.g., during a same imaging session, and the model is trained to generate as output a prediction medical image representing a predicted appearance of the depicted anatomy at a future point in time, e.g., a certain number of years in the future, or with a pathology comprised by the anatomy at a future stage of progression.

One way to do this is to train the prediction model for a specific anatomical region. The model may be configured to generate a prediction medical image representing the anatomy at a pre-defined future time relative to the acquisition time of the input medical image. The pre-defined future time may be referred to as a "prediction interval". The model may be trained, for example, to project the anatomy 5 or 10 years into the future. The output prediction medical image may represent the predicted appearance of the anatomical region after the specified prediction interval.

In alternative embodiments, the prediction model may be trained to receive the prediction interval as a separate input. In such cases, the prediction interval may be provided at inference time, and the model may generate a prediction medical image based on the specified interval. The prediction medical image may depict the anatomical region at a future time corresponding to the received interval.

In further embodiments, the model may be configured to receive a disease progression parameter as an input. The progression parameter may correspond to a future state of a pathology or disease depicted in the input medical image. The model may be trained to generate an output prediction medical image representing the anatomical region affected by the disease at the specified progression state. The appearance of the anatomy in the output prediction medical image may reflect morphological or physiological changes associated with disease progression.

In one example implementation, the prediction model may be implemented using an artificial neural network. A neural network may be provided and trained using a plurality of training data entries. Each training data entry may include an input training image pair.

The input training image pair may comprise a medical image representing a specific anatomical region and an optical image representing an exterior body area corresponding to that region. The medical image and the optical image may be acquired contemporaneously during a single imaging session. Each training data entry may further include a target medical image.

The target medical image may depict the same anatomical region of the same subject as represented in the input training image pair. The target medical image may be acquired at a time point subsequent to the acquisition of the input images. A time interval may separate the acquisition of the input training image pair and the target medical image.

The time interval may correspond to a pre-defined prediction interval. Alternatively, training data entries may include target medical images acquired at varying time intervals relative to the input image pair. The model may thereby be trained to predict anatomical progression over fixed or variable time intervals based on contemporaneously acquired multimodal input data.

Optionally, associated metadata may be included for each image set. This metadata may comprise patient demographic information, clinical information, and image acquisition parameters. Patient demographic information may include age, sex, height, and weight. Clinical information may encompass diagnosis, disease stage, and treatments received.

The neural network architecture may, by way of one example, be implemented as follows.

An encoder network may be employed to process the input medical and optical images. This encoder may include convolutional layers for hierarchical feature extraction, instance normalization and ReLU activation functions, and max pooling layers for downsampling. A latent space may be utilized to combine and process extracted features. This space may contain fully connected layers and employ dropout for regularization. A decoder network may be used to generate the prediction medical image. This decoder may incorporate transposed convolutional layers for upsampling, skip connections from the encoder to preserve fine details, and sigmoid activation in the final layer.

The model may be trained using various techniques. A combination of loss functions may be employed. These may include mean squared error loss between predicted and target medical images, perceptual loss using pre-trained networks to preserve anatomical structure, and adversarial loss from a discriminator network to enhance realism.

During inference, the trained model may accept a new medical image and optical image pair as input. These images may be processed through the encoder and latent space. A prediction medical image may then be generated via the decoder. Post-processing techniques may be applied, such as histogram matching to align intensity distributions and anatomical landmark detection to ensure structural consistency.

The model's performance may continue to be evaluated after training using various methods.

The trained model may be periodically updated with new training data. This updating may serve to improve accuracy and adapt to evolving imaging technologies and patient populations.

With regards to the optical image, in some embodiments, the image prediction module may comprise a dedicated feature extraction layer configured to process the received optical image and extract a predetermined set of one or more features from the image, and wherein the trained image prediction model is configured to process the received medical image and the set of extracted features and to generate as an output the prediction medical image or prediction optical image. In other words, the model is trained to receive the input image and the set of extracted features.

The input to the model relating to the optical image may thus be a set of one or more features extracted from the optical image. These features may include, but are not limited to, skin features, posture, and pose. Additional features that may be extracted may comprise body mass index (BMI) indicators, visible signs of aging, and external manifestations of medical conditions. With regards to skin features that may be extracted, these may include, but are not limited to: texture, color variations, and the presence of lesions or other dermatological conditions. Posture-related features may encompass spinal alignment, shoulder positioning, and overall body stance. Pose features may include limb positioning and body orientation relative to the imaging plane.

The feature extraction process may for example be performed using computer vision techniques. These may include edge detection algorithms, texture analysis methods, and machine learning-based feature detectors.

By utilizing extracted features rather than raw pixel data from the optical image, the model's input dimensionality may be reduced. This reduction may lead to improved computational efficiency.

The specific set of features to be extracted may be predetermined based on the particular medical application and the type of predictions being generated. For instance, in orthopedic applications, posture and skeletal alignment features may be prioritized. In dermatological applications, a greater emphasis may be placed on skin texture and color features.

In some embodiments, the dedicated feature extraction layer may be trainable, allowing it to adapt and optimize the feature extraction process based on the specific requirements of the prediction task. Alternatively, it may utilize pre-defined feature extractors.

In some embodiments, and by way of variation from the simple example described above, the image prediction module may be configured with a multi-channel architecture. This architecture may comprise a trained prediction model with distinct input channels. A first input channel may be configured to handle data from the optical image of the patient's exterior body area. A second input channel may be dedicated to processing data from the medical image of the anatomy.

In this configuration, the model architecture may differ from a single-channel approach. The encoder network may be split into two parallel pathways. Each pathway may be optimized for the respective input modality. The optical image pathway may utilize layers suited for natural image processing. The medical image pathway may employ convolutional layers tailored for radiographic data.

Feature extraction may occur independently in each channel. The optical image channel may emphasize external physical characteristics. The medical image channel may focus on internal anatomical structures. These parallel processes may allow for specialized feature learning.

The extracted features from both channels may then be combined. This combination may occur in a fusion layer or within the latent space. Various fusion strategies may be employed. These could include concatenation, element-wise addition, or more complex attention mechanisms.

The fused representation may then be processed through shared fully connected layers. These layers may learn to correlate features from both modalities. The decoder network may remain similar to the previously described architecture. However, it may now generate predictions based on the combined multi-modal input.

This multi-channel approach may offer several advantages. It may allow for more nuanced predictions by leveraging complementary information. The model may learn to associate external visual cues with internal anatomical changes. This could potentially improve prediction accuracy and robustness.

Training procedures may need to be adapted for the multi-channel model. Separate loss functions may be applied to each input channel during initial stages. A joint loss may then be used to optimize the fusion and subsequent layers. This approach may ensure effective learning from both input modalities.

In accordance with one or more advantageous embodiments, the image prediction model utilized by the image prediction module may comprise a generative adversarial network (GAN) architecture.

GANs consist of two neural networks: a generator and a discriminator. These compete against each other during training. The use of GANs enables the generation of highly realistic prediction images.

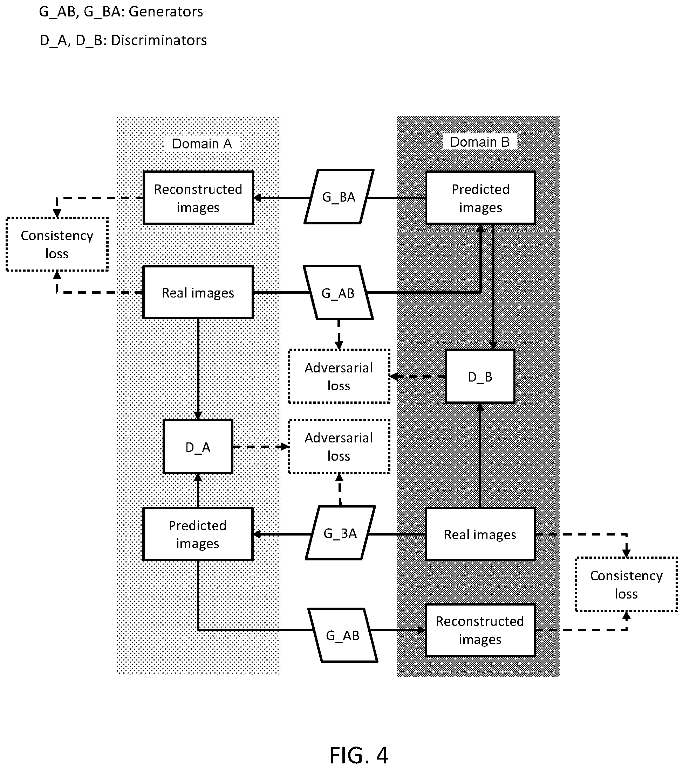

In a preferred embodiment, the image prediction model may be trained using a cycle-GAN operation applied to a training data set as shown in Fig. 4. Cycle-GANs are particularly effective for maintaining consistency between input and output image domains. This helps in preserving anatomical structure in medical image predictions.

A particular advantage of cycle-GAN training is that it does not require paired input-output training images. Instead, it utilizes unpaired images from two different domains. For example, as shown in Fig. 4, one set of real images in domain A and one set of real images in domain B. This characteristic is particularly valuable in medical imaging, where paired future state images are often unavailable. The cycle-GAN training process leverages cycle consistency loss to enforce structural coherence. This mechanism ensures that the generated images maintain fidelity to the original anatomical structures. The cycle consistency principle applies transformations in both forward and backward directions between domains.

The cycle-GAN architecture also enables bidirectional transformations between domains. That is, a transformation of the images in domain A to domain B and vice-versa as shown in Fig. 4. The transformation may be applied to real images and/or to predicted images. This capability allows the model to generate both future predictions from current images and hypothetical current states from predicted future images.

Example GAN architectures suitable for implementation in embodiments of the present invention are described in detail in the following papers:
Pantraki, Evangelia, and Constantine Kotropoulos. "Face aging as image-to-image translation using shared-latent space generative adversarial networks." In 2018 IEEE Global Conference on Signal and Information Processing (GlobalSIP), pp. 306-310. IEEE, 2018.
Liu, Ming-Yu, Thomas Breuel, and Jan Kautz. "Unsupervised image-to-image translation networks." Advances in neural information processing systems 30 (2017).

By way of brief explanation, a suitable GAN architecture model might be implemented as follows. At a high level, the GAN may comprise a first and second generator network G_AB and G_BA and a first and second discriminator network D_A and D_B, as mentioned above and shown in Fig. 4. Each generator network may be structured as an encoder-decoder architecture as discussed in more detail below. The encoder may process the input medical and/or optical image through convolutional layers to extract hierarchical features. These features may be passed through a latent space where they may be combined and processed. The decoder may then generate the prediction medical image using transposed convolutional layers.

Discriminators are typically configured to evaluate the authenticity of both real and predicted image pairs within each domain. Fig. 4 illustrates discriminator D_A for Domain A and discriminator D_B for Domain B. These discriminators aim to distinguish between image pairs comprising real images from their respective domains and image pairs comprising at least one synthetic or predicted image produced by the generators. The output of the discriminator may be used to compute an adversarial loss, which may be incorporated into the generator training objective.

In the single-channel configuration, the input medical image and optical image may be processed sequentially. The medical image may first be input into the generator network's encoder. The encoder may comprise multiple convolutional layers, each followed by batch normalization and ReLU activation functions. Following the processing of the medical image, the optical image may be passed through a dedicated feature extraction layer. This layer may employ computer vision techniques such as edge detection, texture analysis, and color histogram computation to extract relevant features. The extracted features may be encoded into a fixed-length vector representation.

The latent representations from both the medical image encoder and the optical image feature extractor may then be concatenated. This combined representation may be passed through a series of fully connected layers within the latent space. These layers may learn to correlate features from both modalities and may employ dropout for regularization.

The decoder portion of the generator may utilize transposed convolutional layers to progressively upsample the latent representation. The final layer of the decoder may employ an activation function to produce the prediction medical image.

The discriminator network may be implemented as a series of convolutional layers followed by fully connected layers. It may be trained to distinguish between real medical images and those generated by the generator network. The discriminator's output may be used to compute an adversarial loss, which may be incorporated into the generator's training objective.

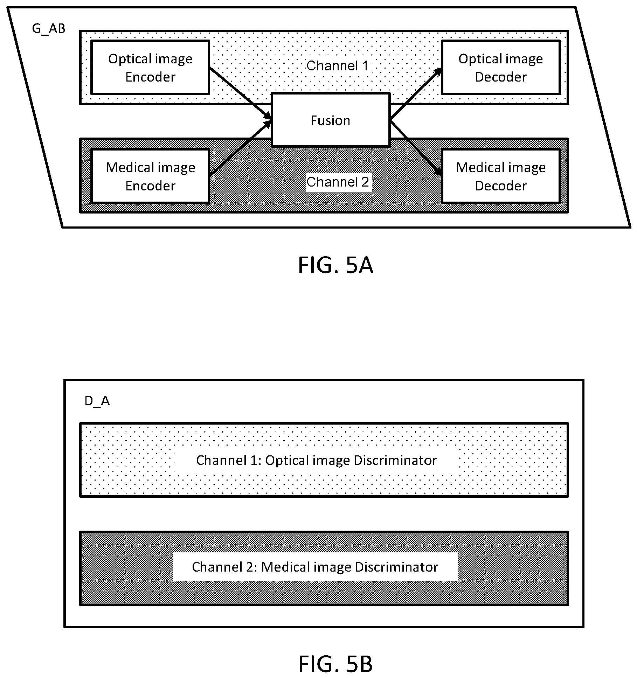

For the multi-channel configuration, the generator's encoder may be split into multiple parallel pathways. In the example shown in Fig. 5A, the generator is split into two parallel pathways. One pathway may process the optical image input, while the other may handle the medical image input. Each pathway may utilize convolutional layers optimized for its respective input modality. The features extracted from both pathways may be combined in a fusion layer or within the latent space. The fusion of features from both modalities may be accomplished through a cross-attention module. This module may allow features from one modality to attend to relevant features in the other modality. The output of the cross-attention module may be passed through a series of transformer blocks to further process the multi-modal representation. The combined features may then be processed through the decoder to generate the prediction medical image.

The discriminator in the multi-channel setup may be adapted to process both medical and optical images. It may evaluate the consistency between the input image pair and the generated prediction image. This evaluation may help ensure that the generated predictions maintain coherence with both input modalities. Alternatively, the discriminator may also be split into parallel channels. For instance, the exemplary discriminator of Fig. 5B is split into two pathways. One pathway for optical images, and one pathway for medical images.

To implement a cycle-consistent GAN (CycleGAN) training process (for either the single channel or multi-channel approach), two generator-discriminator pairs may be utilized as shown in Fig. 4. The first pair may learn to transform images from a first domain to a second domain, for example from the current time point to a future time point or from a current stage of a disease progression to a future stage of a disease progression. The second pair may learn the inverse transformation. Accordingly, the cycle-consistent training process may involve four networks: two generators (G_AB and G_BA) and two discriminators (D_A and D_B) as shown in Fig. 4. Generator G_AB may learn to transform images from the current time point or stage of a disease progression (domain A) to a future time point or stage of a disease progression (domain B), while G_BA may learn the inverse transformation.

During each training iteration, a batch of images (Real images in Fig. 4) may be sampled from both domains. Generator G_AB may produce predicted images in domain B based on input real images from domain A, which discriminator D_B may attempt to distinguish from real domain B images. Similarly, G_BA may generate predicted domain A images based on input real images in domain B, to be evaluated by D_A. The generators may be updated based on a combination of adversarial loss, cycle consistency loss, and identity loss.

The adversarial loss is computed from the adversarial process already described above wherein the discriminator attempts to distinguish between real medical images and those generated by the generator network and wherein the discriminator's output is used to compute the adversarial loss, which may be incorporated into the generator's training objective.

The cycle consistency loss may be computed by passing the predicted images back through the opposite generator (i.e., if the original transformation was through G_AB, the opposite transformation is G_BA) and comparing the result to the original input. This loss may ensure that the learned transformations are invertible and preserve important image characteristics.

The identity loss is an optional additional feature and may be calculated by passing images from one domain through the generator of the same domain (e.g., passing images from domain B through the generator G_AB). This loss may help preserve color and contrast characteristics when appropriate. The relative weights of these loss components may be tuned as hyperparameters during the training process.

The CycleGAN approach may allow for training on unpaired data, where exact matches between current and future time point images are not required. This flexibility may be particularly valuable in medical imaging contexts where paired data may be scarce or difficult to obtain.

By way of brief summary, the training processes outlined above involves repeated determinations of three kinds of loss: (i) Adversarial loss is used in all GANs and measures how well the generator is able to fool the discriminator. The generator aims to minimize this loss, while the discriminator aims to maximize it. In a CycleGAN, there are typically two adversarial losses, one for each direction of transformation. (ii) Cycle consistency loss is specific to CycleGANs and measures how well an image can be reconstructed after being transformed to the other domain and back. For example, if an image x_A is transformed to from domain A to domain B and then back to domain A, the result should be close to the original image x_A. (iii) Identity loss is an optional loss used in some CycleGAN implementations and encourages the generator to act as an identity function when given an image from the target domain. For example, if a generator that transforms from domain A to B is given a B domain image, it should output the same B domain image. This loss can help preserve color and contrast when appropriate.

These losses are typically combined with different weights to form the overall training objective for the CycleGAN. The relative importance of each loss can be adjusted as hyperparameters during the training process.

The GAN training operation applied to train the image prediction model utilizes a training dataset comprising a plurality of training image pairs, that is medical and optical image pairs, at a plurality of different ages, disease progressions, etc. Each training image pair includes a medical image of an anatomy of a patient and an optical image of the exterior area of the body of the same patient. The medical image and optical image in each pair are acquired concurrently during a same imaging session, ensuring temporal consistency between the internal and external representations.

The training dataset includes image pairs corresponding to patients of different ages and/or at different progressions of pathology of the anatomy. This diversity in the training data allows the model to learn patterns associated with both aging and disease progression across different modalities.

During training, the generators and discriminators engage in an adversarial process. The generators attempt to produce increasingly realistic transformed image pairs, while the discriminators become more adept at identifying synthetic pairs. The cycle consistency principle is maintained in this multi-channel setup, ensuring that image pairs transformed from Domain A to Domain B can be accurately transformed back to Domain A.

By processing both medical and optical images concurrently, a single or multi-channel cycle GAN architecture leverages complementary information from internal anatomical structures and external body surfaces. This approach enables the model to take into account both internal changes and external manifestations of aging or disease progression.

Fig. 4 schematically illustrates the training of a cycle-GAN, for the two example generators G_AB, G_BA and two discriminators D_A and D_B. Generator G_AB is being trained to transform images from a current patient age (domain A) to a future patient age (domain B), while G_BA is being trained to perform the inverse transformation. The trained generators may be used as the image prediction models comprised by the image prediction module.

During each training iteration, a batch of images may be sampled from both domains. Generator G_AB generates fake or predicted medical images for a future patient age (domain B), based on an input real medical image and optical image pair for an earlier patient age A. The discriminator D_B attempts to distinguish the fake medical image generated by G_AB from an real medical images for Age B. Similarly, G_BA may generate fake domain A images to be evaluated by D_A.

In some embodiments, the model architecture may be expanded to allow for the model to additionally receive as input non-imaging medical information associated with the patient to which the input medical image and input optical image correspond, such as medical history information and/or values for one or more clinical parameters. The values of one or more clinical parameters may include values for one or more vital signs. The GAN model may be adapted to include one or more additional input channels to accommodate this.

Examples of non-imaging medical information which the model may be trained to receive as input may include one or more of: pose, weight, BMI, injuries, pathologies, prosthetics, cardiac and respiratory cycle information, heart disease status, diabetes status, osteoporosis status, bone density, and osteoarthritis status.

In some examples, the clinical parameters may be derived from one or both of the medical image and optical image pair. This alternative expanded GAN architecture may be configured to process an input set comprising a medical image for a patient, a contemporaneously acquired optical image of the outside of the patient's body and a set of the one or more clinical parameters.

Additionally or alternatively, in some embodiments, the imaging apparatus 40 used to acquire the medical image and the optical image may include a physiological parameter measurement system for measuring one of more physiological parameters, for example including one or more vital signs, while the medical and optical images are being acquired, during the imaging session. The image prediction model may be trained to receive values for the measured one or more physiological parameters as further input in addition to the medical image and the optical image.

The multi-channel GAN model architecture may be adapted to incorporate additional non-imaging clinical parameters through the introduction of a tertiary input channel. This channel may be designed to process a vector of clinical parameters alongside the medical and optical image channels. The clinical parameter vector may be passed through a series of fully connected layers to extract relevant features. These extracted features may then be concatenated or fused with the features derived from the medical and optical image channels. The fusion process may occur within the latent space of the generator network. A cross-attention mechanism may be employed to allow features from the clinical parameters to modulate the image-derived features. The combined representation may subsequently be processed through the decoder portion of the generator to produce the prediction medical image. The discriminator network may also be modified to evaluate the consistency between the input triplet (medical image, optical image, clinical parameters) and the generated prediction image.

An example GAN model has been discussed wherein the two domains A and B correspond to two different ages: Age A and Age B. However, the same principles can be applied to train a model to predict an image in which a pathology is at a future stage of progression. Here the two domains would correspond to two pathology stages, Stage A and Stage B.

Likewise, the examples illustrated in the figures show a model trained to output prediction medical images of the anatomy, but the same principles can be used to train a model to output a prediction optical image representing a predicted appearance of the outside of the patient's body at a future point in time or for the patient with a pathology at a future stage of progression.

Furthermore, in some embodiments, the prediction model may additionally or alternatively be configured to generate multiple prediction images corresponding to different disease stages or time points. Each prediction image may be associated with a corresponding probability value. This functionality allows the model to output a range of potential future appearances for the anatomy or exterior body area. The probability values may indicate the likelihood of each predicted outcome. Multiple prediction images with their associated probabilities can provide clinicians with a more comprehensive view of possible disease progression. This approach may be particularly valuable for conditions with variable progression rates or multiple potential outcomes. The model may be trained on datasets that include multiple future time points or disease stages for each input image pair. During inference, the model may be configured to generate a set of prediction images spanning these different potential futures. In an example, a user may also input a time-frame for the prediction.

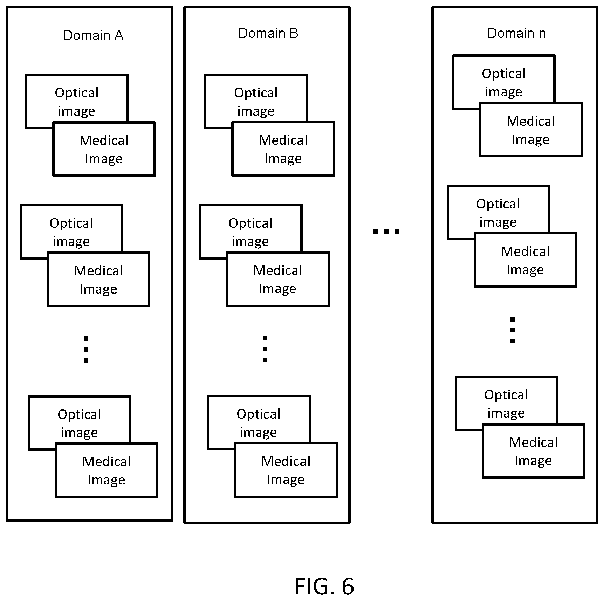

Fig. 6 displays a sample training data set comprising image pairs in a plurality of domains A, B, up to n, wherein n can take any number.

While generative adversarial networks (GANs) represent an advantageous architecture for image prediction tasks, alternative neural network architectures may also be employed. These alternatives may include, but are not limited to, densely connected convolutional networks (DenseNets), U-shaped convolutional networks (U-Nets), and Fourier transform-based neural networks (FNets).

Once trained, the cycleGAN may be used to selectively predict medical and/or optical images comprising a progression or aging prediction at a plurality of stages as comprised in the training data.

In certain embodiments, bias present in existing training datasets may be reduced or mitigated. In particular, age-related bias may be addressed through the generation or selection of medical images that exhibit only specific features typically associated with aging, while omitting others. Features commonly correlated with increasing age in radiographic images may include calcification of cartilage, vascular calcifications (e.g., plaque), narrowing of joint spaces indicative of osteoarthritis, and reductions in bone density. Additional age-associated features may include the presence of bone surface calcifications or joint-adjacent bone enlargements, soft tissue masses, vertebral compression fractures, general bone fractures, disc prolapse, and the presence of implants.

A set of synthesized or selectively curated images may be constructed in which these features are independently varied or selectively excluded. Such a corpus may be configured such that statistical correlations between age-related features are reduced or eliminated. The resulting decorrelated image corpus may enable more specific training or evaluation of the prediction model. In particular, such a dataset may support the detection, staging, or prediction of a target pathological condition independently of unrelated anatomical variations.

The decorrelated corpus may be used as a training dataset for the prediction model described in this disclosure. Alternatively, the prediction model itself may be employed to synthesize medical images representing decorrelated combinations of features. In this way, the prediction model may support the generation of synthetic datasets suitable for unbiased training of other machine learning tools.

In some embodiments, this technique may be used to train models that are more robust to confounding factors such as age, implant presence, or comorbid conditions. Such models may be configured to focus on a particular pathology or disease state while ignoring unrelated anatomical or physiological features.

At least one advantageous application of the invention is for predicting a time to begin or change a therapy for a patient. This potential application is particularly valuable in managing slowly progressing diseases. The prediction model assists a clinician by providing a visual prediction of the progression of anatomical changes in the generated images. Based on this analysis, a clinician can better estimate when certain therapeutic interventions may become necessary.

To augment applicability for this use case, in some embodiments, the training data used to train the prediction model might be annotated with therapy information, i.e. information about therapeutic interventions applied to patients to which the training images relate. Such annotations are typically available in clinical settings. Repeat images are routinely taken to monitor patients and determine therapy initiation or modification. This clinical data provides a source of information for training the prediction model.

By way of example, the prediction model may be trained to output predicted times for future therapy changes. These predictions are based on training data which comprises medical images at different disease progression stages and associated clinical annotations. The model learns to correlate image features with optimal timing for therapeutic interventions.

By incorporating therapy timing predictions, the system may provide additional decision support for clinicians, assisting in planning timing for treatment initiation for newly diagnosed conditions. Additionally, it may assist in determining if and when to modify or escalate existing therapies. Predicting therapy timing can potentially improve patient outcomes. Early intervention, when indicated by the system, may prevent disease progression or complications. Conversely, avoiding premature treatment changes can reduce unnecessary interventions and potential side effects.

One further advantageous application of the invention is for generating training data for other AI models.

Medical image analysis often suffers from dataset biases. For example, positive lung nodule cases are more prevalent in older patients. Conversely, negative chest x-ray findings are more common in younger patients. Such biases can lead machine learning models to overestimate disease likelihood in older patients.

Counteracting these biases typically requires adding more diverse samples. However, obtaining sufficient real-world data can be challenging. Embodiments of the present invention may be used to address this issue by generating synthetic images. Embodiments of the invention may for example be applied to produce x-rays of healthy older patients or diseased younger patients. This capability enables the creation of balanced, bias-free training datasets for other AI models.

By generating synthetic images of healthy older patients or diseased younger patients, the system addresses common biases in medical image datasets. These biases often result from the natural correlation between age and certain medical conditions in real patient populations.

The ability to create balanced datasets is particularly valuable for rare diseases or uncommon combinations of age and pathology. The system can generate synthetic examples that may be difficult or impossible to obtain from real patient data. This approach allows for the creation of large, diverse training datasets without the need for extensive patient recruitment or data collection. It enables the development of more robust and unbiased AI models for medical image analysis. The synthetic images can be used to augment existing datasets, improving the representation of underrepresented groups or conditions.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method (10) comprising:
receiving (12) a medical image (72) of an anatomy of a patient;
receiving (12) an optical image (74) of an exterior area of the patient's body;
inputting (16) the medical image and the optical image into an image prediction module (80) comprising a trained prediction model, responsive to which, outputting (20) from the image prediction module either or both of:
a prediction medical image (22) representing a predicted appearance of the anatomy;
a prediction optical image (24) representing a predicted appearance of the exterior area of the patient's body.

2. The method of claim 1, wherein the exterior area of the patient's body corresponds to an exterior of an area of the body which contains the said anatomy.

3. The method of any preceding claim, wherein
the anatomy of the patient represented in the received medical image is a pathology at a first stage of progression.

4. The method of claim 3, wherein:
the prediction medical image represents a predicted appearance of said anatomy of the patient with the pathology at a later stage of progression; and/or
the prediction optical image represents a predicted appearance of the exterior area of the patient's body with the pathology at a later stage of progression.

5. The method of any preceding claim, wherein:
the prediction medical image represents a predicted appearance of said anatomy of the patient at a later point in time; and/or
the prediction optical image represents a predicted appearance of the exterior area of the patient's body at a later point in time.

6. The method of any preceding claim,
wherein the image prediction module comprises a feature extraction layer configured to process the received optical image and extract a predetermined set of one or more features from the image, and
wherein the trained image prediction model is configured to process the received medical image and the set of extracted features and to generate as an output the prediction medical image or prediction optical image.

7. The method of any preceding claim,
wherein the image prediction model comprises a generative adversarial network, GAN, and
optionally wherein the image prediction model is trained using a cycle-GAN operation applied to a training data set.

8. The method of claim 7,
wherein the image prediction model is trained using a cycle-GAN operation applied to a training data set, and
wherein the cycle-GAN operation is applied using a training dataset comprising a plurality of training image pairs, wherein each training image pair comprises a medical image of said anatomy of a patient and an optical image of the exterior area of the body of the same patient, and wherein the medical image and optical image are images acquired concurrently in a same imaging session.

9. The method of claim 8, wherein the plurality of training image pairs include image pairs corresponding to patients of different ages and/or at different progressions of pathology of the anatomy.

10. The method of any preceding claim, wherein the trained prediction model is a multi-channel model having a first input channel and a second input channel, and wherein
the first input channel is configured to receive and process data corresponding to the optical image of the exterior area of the patient's body, and
the second input channel is configured to receive and process data corresponding to the medical image of the anatomy.

11. The method of any preceding claim, wherein the received medical image is any one of: an x-ray image, a computed tomography (CT) image, a magnetic resonance (MR) image, or an ultrasound image.

12. The method of any preceding claim, wherein the predicted medical image depicts a predicted appearance of an image of the anatomy captured with an imaging modality which is the same as the imaging modality of the received medical image.

13. A computer program product comprising computer program code configured, when run on a processor, to perform a method in accordance with any preceding claim.

14. A processing device (32) comprising one or more processors (36) configured to perform a method in accordance with any of claims 1-12.

15. A system (30) comprising:
an imaging apparatus (40) comprising: a medical imaging system (50), an optical imaging system (60) and an imaging controller (42) operatively coupled to the medical imaging system and the optical imaging system, wherein the imaging controller is configured to control acquisition of a medical image (72) of the anatomy of the patient and an optical image (74) of the exterior area of the body of the patient during a same imaging session; and
a processing device (32) as claimed in claim 14.
